# DEMANDE DE BREVET EUROPEEN

(11) **EP 4 360 611 A1**
(43) Date de publication de la demande: **01.05.2024**
(21) Numéro de dépôt: 23205696.0
(22) Date de dépôt: 25.10.2023
(51) Int. Cl.: A61K 8/02, A61K 8/44, A61Q 19/08

(54) **COMPOSITION DERMATOLOGIQUE COMPRENANT DES PARTICULES DE L-GLUTAMINE**

(30) Priorité: 26.10.2022 BE 202205873
(71) Demandeur: Alphascience Research, 1410 Waterloo (BE)
(72) Inventeur: MARCHAL, Alfred, 1410 Waterloo (BE)
(74) Mandataire: Gevers Patents

(57) **Abrégé**

La présente demande de brevet concerne une composition dermatologique destinée à être utilisée par administration topique, ainsi que l'utilisation non-thérapeutique de ladite composition pour favoriser la densification et/ou l'homogénéisation de l'épiderme, atténuer les rides, atténuer les vergetures, repulper et/ou raffermir la peau.

## Description

### Domaine de l'invention

La présente demande de brevet concerne une composition dermatologique destinée à être utilisée par administration topique, ainsi que l'utilisation non-thérapeutique de ladite composition pour favoriser la densification et/ou l'homogénéisation de l'épiderme, atténuer les rides, atténuer les vergetures, repulper et/ou raffermir la peau.

### Arrière-plan technologique

La glutamine (L-glutamine) est l'acide aminé le plus abondant sous forme libre dans le plasma humain. Bien qu'étant classifié comme un acide aminé non-essentiel, la glutamine peut le devenir dans des conditions de stress sévère dans lesquelles les concentrations intracellulaires et plasmatiques de glutamine peuvent diminuer de plus de 50% et 30%, respectivement (Kovacevic Z., McGivan J. Mitochondrial metabolism of glutamine and glutamate and its physiological significance. Physiol Rev. 1983 ; 63 : 547-605).

Cependant, contrairement aux autres acides aminés, la glutamine est instable en solution, ce qui limite son utilisation dans des formulations topiques, qu'elles soient à vocation cosmétiques ou thérapeutiques.

La glutamine est entre autre connue pour jouer un rôle important dans le traitement des affections dermatologiques, et participe notamment à la synthèse du collagène. Dans ce contexte, la glutamine accélère et favorise la restructuration des cellules de la peau lorsqu'elles sont endommagées, assurant ainsi sa bonne santé.

La glutamine est en particulier utilisée dans le traitement et la prévention des rides. Les rides sont des plis, crêtes ou crevasses dans la peau qui apparaissaient naturellement avec l'âge. Les rides plus généralement marqués sur les peaux exposées au soleil, comme le visage, le cou, les mains ou les avant-bras. De nombreux facteurs influencent l'apparition des rides, notamment l'exposition au soleil, le tabagisme, la déshydratation, certains médicaments et des facteurs environnementaux et génétiques. Le lumière UV décompose les fibres de collagène et d'élastine qui forment le tissu conjonctif qui soutient la peau. A mesure que cette couche se décompose, la peau devient moins flexible, s'affaisse et les rides apparaissent.

Typiquement, la L-glutamine utilisée dans les formulations à vocation médicale ou de laboratoire se présente sous la forme de particules dont la taille volumique médiane est d'environ 25 microns. Cependant, sous cette forme, la L-glutamine présente une faible stabilité et doit donc être formulée à des concentrations élevées.

Il existe donc un besoin pour la mise à disposition de compositions comprenant de la glutamine présentant une durée de conservation étendue ainsi qu'une action dermatologique améliorée, en particulier contre les rides, de manière à permettre la préparation de formulations cosmétiques et thérapeutiques acceptables pour l'industrie. Par ailleurs, ces nouvelles formes stabilisées doivent également pouvoir être produites de façon simple et à un coût raisonnable.

### Résumé de l'invention

Les inventeurs ont découvert de manière surprenante que la présente invention répond aux problèmes mentionnés ci-dessus.

Selon un premier aspect, la présente invention concerne une composition dermatologique destinée à être utilisée par administration topique [composition (D), ci-après] comprenant une quantité dermatologiquement active de particules de L-glutamine ou l'un de ses sels pharmaceutiquement acceptables, et au moins un excipient acceptable pour une administration topique, dans laquelle la taille volumique médiane des particules de L-glutamine ou l'un de ses sels pharmaceutiquement acceptables est inférieure à 15,0 microns.

En d'autres termes, la présente invention concerne une composition dermatologique pour une administration topique [composition (D), ci-après] comprenant une quantité dermatologiquement active de particules de L-glutamine ou l'un de ses sels pharmaceutiquement acceptables, et au moins un excipient acceptable pour une administration topique, dans laquelle la taille volumique médiane des particules de L-glutamine ou l'un de ses sels pharmaceutiquement acceptables est inférieure à 15,0 microns.

Selon un autre aspect, la présente invention concerne également l'utilisation non-thérapeutique de la composition (D) pour favoriser la densification et/ou l'homogénéisation de l'épiderme, atténuer les rides, atténuer les vergetures, repulper et/ou raffermir la peau.

Selon un autre aspect, la présente invention concerne également un procédé de traitement cosmétique de la peau destiné à favoriser la densification et/ou l'homogénéisation de l'épiderme, atténuer les rides, atténuer les vergetures, repulper et/ou raffermir la peau comprenant l'application topique de la composition (D).

### Description détaillée de l'invention

Le terme "comprenant", tel qu'utilisé dans le contexte de l'invention, ne doit pas être interprété comme étant limité aux moyens énumérés par après ; il n'exclut pas la présence d'autres éléments ou étapes. Il doit être interprété comme précisant la présence des caractéristiques énumérées, les éléments, étapes ou composants auxquels il est fait référence, mais n'exclut pas la présence ou l'ajout d'une ou plusieurs autres caractéristiques, éléments, étapes, ou composants, ou des groupes de ceux-ci. Ainsi, la portée de l'expression « une composition comprenant A et B » ne doit pas être limitée à la composition composée uniquement A et B. Cela signifie que, dans le contexte de la présente invention, les seuls éléments pertinents sont A et B. En conséquence, les termes « comprenant » et « incluant » englobent les termes plus restrictifs que sont « consistant essentiellement de » et « consistant en ».

Dans le contexte de la présente invention, les termes « glutamine » et « L-glutamine » sont utilisés de manière interchangeable pour désigner l'acide aminé alpha utilisé dans la biosynthèse des protéines.

Dans le contexte de la présente invention, le terme « sujet » est utilisé pour désigner ici un mammifère, de préférence un humain.

La présente invention concerne une composition dermatologique destinée à être utilisée par administration topique [composition (D), ci-après] comprenant une quantité dermatologiquement active de particules de L-glutamine ou l'un de ses sels pharmaceutiquement acceptables, et au moins un excipient acceptable pour une administration topique, dans laquelle la taille volumique médiane des particules de L-glutamine ou l'un de ses sels pharmaceutiquement acceptables est inférieure à 15,0 microns.

En d'autres termes, la présente invention concerne une composition dermatologique pour une administration topique [composition (D), ci-après] comprenant une quantité dermatologiquement active de particules de L-glutamine ou l'un de ses sels pharmaceutiquement acceptables, et au moins un excipient acceptable pour une administration topique, dans laquelle la taille volumique médiane des particules de L-glutamine ou l'un de ses sels pharmaceutiquement acceptables est inférieure à 15,0 microns.

Les chercheurs ont découvert de manière surprenante que les particules de L-glutamine (glutamine) ou l'un de ses sels pharmaceutiquement acceptables ayant une taille volumique médiane inférieure à 15 microns présentent non-seulement une activité dermatologique supérieure à celle des poudres brutes (non-micronisées) de la glutamine, mais permettent par ailleurs formulation de la glutamine dans des préparations cosmétiques présentant une durée de conservation étendue nécessaire pour des usages industriels.

Comme décrit ci-dessus, la composition (D) comprend une quantité dermatologiquement active de particules de L-glutamine ou l'un de ses sels pharmaceutiquement acceptables dont la taille volumique médiane est inférieure à 15 microns.

En général, la taille volumique médiane des particules peut être mesurée par des méthodes connues dans l'art, par exemple par des analyseurs de taille de particules, des méthodes utilisant la lumière (méthodes de diffusion de la lumière ou méthodes turbidimétriques), des méthodes de sédimentation (analyse à la pipette d'Andreassen, échelles de sémientation, photosédimentomètres ou sédimentation par centrifugation), des méthodes par impulsion (compteur Coulter), ou le tri par la force gravitationnelle ou centrifuge.

En particulier, la taille volumique médiane des particules peut être mesurée en utilisant un appareillage de type Mastersizer 2000 (Malvern Instruments, Inc., Southborough, Mass.).

Dans le contexte de la présente invention, les termes « taille volumique médiane », « diamètre médian » et « D50 » sont utilisés de manière interchangeable pour faire référence à la taille au-dessus ou en dessous de laquelle 50% des particules résident sur une base volumique. Par exemple, pour un échantillon ayant une taille volumique médiane (D50) de 5 microns, correspond à un échantillon dont 50 % des particules sont plus grandes que 5 microns et 50 % des particules sont plus petites que 5 microns.

De préférence, ladite composition (D) comprend une quantité dermatologiquement active de particules de L-glutamine ou l'un de ses sels pharmaceutiquement acceptables dont la taille volumique médiane est inférieure à 13,0 microns, préférentiellement inférieure à 11,0 microns, de manière préférée inférieure à 10,0 microns.

De préférence, ladite composition (D) comprend une quantité dermatologiquement active de particules de L-glutamine ou l'un de ses sels pharmaceutiquement acceptables dont la taille volumique médiane est supérieure à 0,5 microns, préférentiellement supérieure à 1,0 microns, de manière préférée supérieure à 1,5 microns.

Selon une variante préférée de la présente invention, ladite composition (D) comprend, une quantité dermatologiquement active de particules de L-glutamine ou l'un de ses sels pharmaceutiquement acceptables dont la taille volumique médiane est supérieure à 0,5 microns et inférieure à 13,0 microns, préférentiellement supérieure à 1,0 microns et inférieure à 11,0 microns, de manière préférée supérieure à 1,5 microns et inférieure à 10,0 microns.

Selon l'invention, ladite composition (D) comprend une quantité dermatologiquement active de particules de L-glutamine ou l'un de ses sels pharmaceutiquement acceptables.

Dans le contexte de la présente invention, le terme « quantité dermatologiquement active » désigne la quantité d'un ingrédient dermatologiquement actif qui va éliciter la réponse cosmétique au niveau de la peau d'un sujet, animal ou humain, telle que recherchée par un chercheur, un médecin, un vétérinaire ou un autre intervenant clinique ou esthétique (par exemple, la réduction des rides).

Dans le contexte de la présente invention, le terme « sel pharmaceutiquement acceptable » fait référence aux sels d'acide inorganiques ou organiques relativement non-toxiques de la glutamine. Ces sels peuvent être préparés in situ durant la préparation ou l'isolation de la glutamine, ou en faisant réagir séparément la glutamine purifiée avec un acide organique ou inorganique adapté, et en isolant le sel ainsi formé. Des exemples de sels pharmaceutiquement acceptables comprennent les sels hydrobromide, hydrochloride, sulfate, bisulfate, phosphate, nitrate, acétate, valérate, oxalate, benzoate, lactate, phosphate, tosylate, citrate, fumarate, tartrate, succinate.

De préférence, ladite composition (D) comprend, relatif au poids total de la composition (D), au moins 0,5 % en poids, préférentiellement au moins, 1,0 % en poids, encore plus préférentiellement au moins 1,5 % en poids, de manière préférée au moins 2,0 % en poids de particules de L-glutamine ou l'un de ses sels pharmaceutiquement acceptables.

De préférence, ladite composition (D) comprend, relatif au poids total de la composition (D), au plus 10,0% en poids, préférentiellement au plus 8,0% en poids, encore plus préférentiellement au plus 6,0% en poids, de manière préférée au plus 5,0% en poids de particules de L-glutamine ou l'un de ses sels pharmaceutiquement acceptables.

Selon une variante préférée de la présente invention, ladite composition (D) comprend, relatif au poids total de la composition (D), au moins 0,5 % en poids et au plus 10,0 % en poids, préférentiellement au moins 1,0 % en poids et au plus 8,0 % en poids, encore plus préférentiellement au moins 1,5 % en poids et au plus 6,0 % en poids, de manière préférée au moins 2,0 % en poids et au plus 5,0 % en poids de particules de L-glutamine ou l'un de ses sels pharmaceutiquement acceptables.

Selon l'invention, ladite composition (D) comprend au moins un excipient acceptable pour administration topique.

Dans le contexte de la présente invention le terme « acceptable pour administration topique » fait référence au fait que l'excipient présent dans la composition selon l'invention doit (i) être adapté pour être en contact avec la peau d'un sujet sans induire de toxicité injustifiée, d'incompatibilité, d'instabilité, de réponse allergique ou autre, et doit par ailleurs (ii) permettre l'application d'une composition selon l'invention sur la peau et/ou à la surface des membranes muqueuses.

Les excipients offrent à la composition (D) selon l'invention des propriétés physiques et (bio-)chimiques optimales pour une administration topique, lui permettant ainsi d'acquérir certaines propriétés bénéfiques pour obtenir les effets cosmétiques recherchés.

De manière avantageuse, la composition (D) selon l'invention comprend plusieurs excipients acceptables pour une administration topique.

De préférence, ledit au moins un excipient acceptable pour administration topique comprend l'acide oléique.

Les inventeurs ont découvert que la présence d'acide oléique dans la composition (D) permet d'accélérer la pénétration des particules de glutamine dans la peau et/ou les membranes muqueuses des sujets traités. De ce fait, la présence d'acide oléique augmente l'activité dermatologique de la composition (D).

De préférence, ladite composition (D) comprend, relatif au poids total de la composition (D), au moins 2,0 % en poids, préférentiellement au moins 3,0 % en poids, encore plus préférentiellement au moins 4,0 % en poids, de manière préférée au moins 5,0 % en poids de d'acide oléique.

De préférence, ladite composition (D) comprend, relatif au poids total de la composition (D), au plus 20,0 % en poids, préférentiellement au plus 17,0 % en poids, encore plus préférentiellement au plus 15,0 % en poids, de manière préférée au plus 12,0 % en poids d'acide oléique.

Selon une variante préférée de la présente invention, ladite composition (D) comprend, relatif au poids total de la composition (D), au moins 2,0 % en poids et au plus 20,0 % en poids, préférentiellement au moins 3,0 % en poids et au plus 17,0 % en poids, encore plus préférentiellement au moins 4,0 % en poids et au plus 15,0 % en poids, de manière préférée au moins 5,0 % en poids et au plus 12,0 % en poids d'acide oléique.

Selon une variante préférée de l'invention, ladite composition (D) comprend, relatif au poids total de la composition (D), au moins 0,5 % en poids et au plus 10,0 % en poids de particules de L-glutamine ou l'un de ses sels pharmaceutiquement acceptables, et au moins un excipient acceptable pour une administration topique, dans laquelle la taille volumique médiane des particules de L-glutamine ou l'un de ses sels pharmaceutiquement acceptables est inférieure à 15,0 microns, et dans laquelle ledit au moins un excipient acceptable pour une administration topique comprend relatif au poids total de la composition (D), au moins 2,0 % en poids et au plus 20,0 % en poids d'acide oléique.

De manière avantageuse, ladite composition (D) comprend en outre, relatif au poids total de ladite composition (D), de 5,0 à 50,0 % en poids, plus avantageusement de 10,0 à 40,0 % en poids, encore plus avantageusement de 15,0 % à 30,0 % en poids d'un épaississant. Avantageusement, ledit épaississant est la glycérine.

De manière avantageuse, la composition (D) selon la présente invention comprend en outre un agent solubilisant. L'agent solubilisant selon l'invention permet d'augmenter la bioaccessibilité des ingrédients actifs présents dans la composition. Un tel agent solubilisant est avantageusement solvant organique polaire et miscible dans l'eau, de préférence un éther de glycol, de manière encore plus préférée l'agent humectant est le diethylène glycol monoéthyléther (DEGEE). Avantageusement, on utilisera dans le cadre de la présente invention une agent solubilisant tel que le produit connu sous la dénomination commerciale Transcutol^{®} CG. De préférence, ladite composition comprend de 1,0 à 10,0 % en poids dudit agent solubilisant, plus avantageusement de 1,5 à 5,0 %, encore plus avantageusement de en poids 2,0 à 4,0 % dudit agent de solubilisation relatif au poids total de ladite composition (D).

De manière avantageuse, ladite composition (D) comprend en outre, relatif au poids total de ladite composition (D), de 1,0 à 20,0 % en poids, plus avantageusement de 3,0 à 15,0 % en poids, encore plus avantageusement de 5,0 % à 10,0 % en poids d'au moins un agent émolliant. Avantageusement, on utilisera dans le cadre de la présente invention un agent émolliant tel que celui connu sous la dénomination commerciale Emulium^{®} Delta et/ou sous la dénomination commerciale DUB^{®} 810 C.

De manière avantageuse, ladite composition (D) comprend en outre, relatif au poids total de ladite composition (D), de 0,1 à 5,0 % en poids, plus avantageusement de 0,2 à 3,0 % en poids, encore plus avantageusement de 0,5 % à 2,0 % en poids d'une microfibre cellulosique. Avantageusement, on utilisera dans le cadre de la présente invention une microfibre cellulosique telle que celle connue sous la dénomination commerciale Betafib^{®} ETD.

De manière avantageuse la composition (D) selon l'invention comprend un agent antimicrobien. L'agent antibactérien selon l'invention permet d'éviter le développement de contaminations bactériennes et fongiques dans la composition. Un tel agent antimicrobien est avantageusement un mélange d'origine végétale comprenant du pentylène glycol. Avantageusement, on utilisera dans le cadre de la présente invention une agent antimicrobien tel que le produit connu sous la dénomination commerciale E-Leen^{®} Green C. De préférence, ladite composition comprend de 0,1 à 10,0 % en poids dudit agent antibactérien, plus avantageusement de 0.2 à 5.0 %, encore plus avantageusement de 0,5 à 3,0 % en poids dudit agent de antimicrobien relatif au poids total de ladite composition.

De manière avantageuse la composition (D) selon l'invention comprend de l'acide hyaluronique ou l'un de ses sels. L'acide hyaluronique ou l'un de ses sels selon l'invention présente de nombreux bénéfices, notamment pour l'hydratation et le cohésion des tissus, le contrôle de l'angiogenèse, des processus inflammatoires ainsi que de la cicatrisation. De préférence, l'acide hyaluronique ou l'un de ses sels selon l'invention est l'hyaluronate de sodium. Avantageusement, la composition (D) selon l'invention comprend au moins deux types d'hyaluronate de sodium ayant des poids moléculaires différents. De préférence, la composition (D) selon l'invention comprend de l'hyaluronate de sodium ayant un poids moléculaire moyen d'environ 50 kDa ainsi que de l'hyaluronate de sodium ayant un poids moléculaire moyen d'environ 300 kDa. De préférence, ladite composition (D) comprend de 0,01 à 2 % en poids dudit acide hyaluronique ou l'un de ses sels, plus avantageusement de 0,05 à 1 %, encore plus avantageusement de 0,1 à 0,5% en poids dudit acide hyaluronique ou l'un de ses sels relatif au poids total de ladite composition.

De manière avantageuse, ladite composition (D) comprend en outre, relatif au poids total de ladite composition (D), de 20,0 à 70,0 % en poids, plus avantageusement de 30,0 à 60,0 % en poids, encore plus avantageusement de 40,0 % à 55,0 % en poids d'eau.

La composition (D) selon l'invention peut être sous forme solide ou sous forme liquide. De préférence ladite composition est sous la forme d'une solution, d'un gel, d'une lotion, d'une crème, d'un onguent, d'une mousse, d'une émulsion, d'une microémulsion, d'un lait, d'un sérum, d'un aérosol, d'une pulvérisation, d'une dispersion ou d'un savon. De manière encore plus préférée, la composition (D) selon l'invention est sous forme d'une crème.

Dans un autre aspect, la présente invention concerne l'utilisation non-thérapeutique, cosmétique, de la composition (D) selon l'invention pour favoriser la densification et/ou l'homogénéisation de l'épiderme, atténuer les rides, atténuer les vergetures, repulper et/ou raffermir la peau.

Les inventeurs on en effet découvert de manière surprenante que l'apparence générale d'un sujet peut être améliorée suite à l'utilisation de la composition (D) selon l'invention.

La composition (D) selon l'invention peut être par exemple être utilisée pour réduire la profondeur des rides.

Selon un autre aspect, la présente invention concerne également un procédé de traitement cosmétique de la peau destiné à favoriser la densification et/ou l'homogénéisation de l'épiderme, atténuer les rides, atténuer les vergetures, repulper et/ou raffermir la peau, comprenant l'administration topique de la composition de dépigmentation (D), à un sujet en ayant besoin.

L'ensemble des définitions, préférences et aspects préférés décrits ci-dessus pour la composition (D) selon l'invention s'appliquent également *mutatis mutandis* pour son utilisation non-thérapeutique pour favoriser la densification et/ou l'homogénéisation de l'épiderme, atténuer les rides, atténuer les vergetures, repulper et/ou raffermir la peau.

L'invention va à présent être décrite plus en détail en se référant aux exemples suivants, qui détaillent des illustrations non-limitatives de différents aspects de l'invention.

### Exemples

### Exemple 1: Préparation d'une composition dermatologique (D) selon l'invention.

Une composition dermatologique (D) selon l'invention a été préparée en mélangeant les différents ingrédients repris dans le Tableau 1, ci-dessous. La formulation de la composition est détaillée ci-dessous et se présente sous la forme d'une crème.

**Tableau 1**

| **Ingrédients de la composition (D)** | **Pourcentage en poids relatif au poids total de la composition (D)** |
|---|---|
| Glutamine micronisée | 3,00 |
| Emulium^{®} Delta | 6,00 |
| Acide oléique | 9,00 |
| DUB 810 C | 3,00 |
| Betafib ^{®} ETD | 0,75 |
| Glycérine | 22,00 |
| Transcutol^{®} CG | 2,00 |
| E-Leen^{®} Green C | 1,50 |
| Eau déminéralisée | 52,75 |
| **Total** | **100** |

Une analyse granulométrique de l'échantillon de glutamine micronisée utilisé pour la préparation de la composition (D) détaillée dans le Tableau 1 a été réalisée et les résultats ont été comparés avec ceux obtenus pour un échantillon de poudre de glutamine brute (non-micronisé). Les spécifications sont reprises dans le Tableau 2, ci-dessous.

**Tableau 2**

| **Caractéristiques (µm)** | **Glutamine micronisée** | | **Glutamine poudre brute (non-micronisée)** |
|---|---|---|---|
| | **Test 1** | **Test 2** | |
| D10 | 0,58 | 1,24 | 3,99 |
| D50 | 2,80 | 7,61 | 25,75 |
| D90 | 6,84 | 17,92 | 74,13 |
| D95 | 8,16 | 21,19 | 99,98 |
| D99 | 10,31 | 26,39 | 151,39 |
| D100 | 13,20 | 31,31 | 205,08 |

Les mesures ont été réalisées à l'aide d'un appareillage Mastersizer 2000 (Malvern Instruments, Inc., Southborough, Mass.).

Le D50 est également appelé diamètre médian des particules ou taille volumique médiane des particules. Par exemple, un échantillon ayant une valeur de D50 de 5 µm, correspond à un échantillon dont 50 % des particules sont plus grandes que 5 µm et 50 % des particules sont plus petites que 5 µm. De mêmes, les valeurs D10, D90, D95, D99 et D100 correspondent respectivement aux tailles des particules correspondantes lorsque leur pourcentage cumulé atteint 10 %, 90 %, 95 %, 99 % et 100% respectivement.

### Exemple 2: Evaluation de la stabilité de la glutamine micronisée dans la composition (D) selon l'invention.

La stabilité de la glutamine micronisée dans la composition (D) décrite à l'Exemple 1 a été évaluée sur une période de 6 mois.

Une courbe de calibration a été réalisée en dissolvant de la glutamine dans de l'eau milliQ à des concentrations de 0,1, 0,4, 1,0, 2,0 et 3,0 mg/mL. La courbe de calibration a été réalisée à partir de ces quatre points en rapportant les aires sous les pics mesurés en HPLC à 215 nm en fonction de la masse de glutamine injectée sur la colonne HPLC et des concentrations des standards respectivement. Chaque solution standard a été analysée trois fois en HPLC afin de réaliser une moyenne sur les intégrations de l'aire sous le signal correspondant à la glutamine.

Une échantillon de la composition (D) de 83,3 mg a été pesé dans un ballon de 10 mL et ensuite dilué dans 5 mL d'eau milliQ. La solution aqueuse obtenue est transférée dans une ampoule à décanter et la phase aqueuse est lavée deux fois avec 2 mL d'hexane. La phase aqueuse est récupérée et centrifugée 10 minutes à 4,500 rpm. Finalement, 1 mL de la solution centrifugée est placée dans un pilulier pour analyse HPLC.

Les conditions de l'analyse sont les suivantes :
- Appareillage : HPLC Agilent 1100 series connectée à un détecteur DAD
- Colonne : Lichrosphere 100 RP 18^{e} - paticules 5 µm - 4,0 x 250 mm
- Flux : 1,1 mL/min
- Volume d'injection : 2 µL
- Eluant : MeOH/H₂O - 60/40 (v/v)
- Température colonne : 40°C
- Longueurs d'onde sélectionnées : 204 nm et 254 nm

La teneur en glutamine dans la composition (D) a été évaluée sur une période de 6 mois. A cet effet, la quantité de glutamine a été dosée à intervalles réguliers. La stabilité a été évaluée dans des conditions de conservation normales (25°C et 60% d'humidité relative).

Les résultats de ces expériences sont présentés dans le Tableau 3, ci-dessous.

**Tableau 3**

| **Durée de conservation (mois)** | **Dosage de L-glutamine (% en poids relatif au poids total de la composition (D))** |
|---|---|
| 0 | 8,00 |
| 2 | 6,31 |
| 4 | 6,30 |
| 6 | 6,31 |

Les résultats obtenus démontrent que la composition (D) selon l'invention présente une quantité de glutamine conforme (> 75%) sur l'ensemble de la durée de l'étude de stabilité, soit 6 mois dans des conditions de commerce. Par ailleurs, sur l'ensemble de la durée du test aucune altération de la composition (D) n'a pu être observée. Ces observations sont surprenantes étant donné que la glutamine est généralement dégradée en quelques jours dans des formulations cosmétiques de ce type.

Exemple 3: Evaluation clinique de l'activité de la composition (D) selon l'invention sur les rides.

Ces expériences visent à évaluer l'efficacité sur le traitement des rides d'une application topique de la composition (D) telle que détaillée dans le Tableau 1, ci-dessus.

Cet essai clinique hémifacial est réalisé chez 12 sujets volontaires âgés de plus de 25 ans, de sexe féminin, de phototype 1 à 4. Les sujets n'utilisent pas d'autre traitement anti-rides sur les zones traitées, ni avant ni durant l'étude.

Les critères d'exclusion sont la grossesse, la sensibilité à certains des ingrédients actifs, l'utilisation de produits avec un effet potentiel anti-rides, des affections cutanées du visage (infections de la peau, eczéma, psoriasis, rosacée, herpès, etc.) ou les allergies sévères.

Les sujets reçoivent un tube contenant la composition (D) sous forme de crème. La crème est appliquée chaque matin et chaque soir sur les rides. La quantité de produit est ajustée pour une bonne pénétration cutanée. La durée du traitement est de 8 semaines. Les surfaces traitées sont les pattes d'oies (fines lignes autour des yeux) et les rides péribuccales. Des photos sont prises avant, pendant et après le traitement. Les mesures sont réalisées par emprunte au silicone de manière à évaluer et comparer la profondeur des rides avant et durant l'application de la crème. De cette manière, une évaluation subjective ainsi qu'une mesure objective de la profondeur des rides par emprunte au silicone est réalisée.

L'évaluation des sujets est à la fois subjective (sujets) et objective (médecin). Les sujets sont évalués 0, 4 et 8 semaines après le début du traitement. Le médecin utilise un profondimètre. Une évaluation numérique est réalisée selon l'échelle suivante :
0 = pas d'amélioration
1 = amélioration modérée
2 = amélioration marquée
3 = disparition des rides

Le médecin évalue également les effets secondaires possibles du traitement.

L'ensemble des données récoltées démontrent que le traitement avec la composition (D) diminue les rides (diminution de la profondeur des rides), et ce sans effet secondaire.

### Exemple 4: Essais comparatifs

### Essai comparatif 1

Un essai clinique comparatif a été réalisé afin de comparer les activités / les efficacités respectives sur les rides des compositions suivantes :
a) composition selon l'invention telle que mentionnée au Tableau 1 dans laquelle la L-glutamine est présente sous forme de particules ayant une taille volumique médiane inférieure à 15,0 microns;
b) composition telle que mentionnée au Tableau 1 dans laquelle la L-glutamine est présente sous forme cristalline ayant une taille volumique médiane de 154 microns.

Pour chacune des compositions a) et b) décrites ci-dessus, l'essai clinique comparatif a été réalisé selon le protocole décrit à l'Exemple 3.

Les résultats obtenus ont démontré qu'une application d'une composition a) selon l'invention comprenant des particules de L-glutamine ayant une taille volumique médiane inférieure à 15,0 microns donne lieu à une diminution significativement augmentée des rides en comparaison avec une composition b) comprenant de la L-glutamine sous forme cristalline ayant une taille volumique médiane de 154 microns.

### Essai comparatif 2

Des compositions ont été formulées comme indiqué au tableau 4 ci-après et ont été évaluées en termes de texture et de stabilité de la forme galénique.

**Tableau 4**

| | **Composition selon l'invention** L-glutamine **micronisée** : taille volumique médiane des particules de L-glutamine inférieure à 15,0 microns (pourcentage en poids relatif au poids total de la composition) | **Composition comparative** L-glutamine **cristalline** : taille volumique médiane des particules de L-glutamine de 154 microns (pourcentage en poids relatif au poids total de la composition) |
|---|---|---|
| L-glutamine | 3,00 | 3,00 |
| Emulium^{®} Delta | 6,00 | 6,00 |
| Acide oléique | 9,00 | 9,00 |
| DUB 810 C | 3,00 | 3,00 |
| Betafib ^{®} ETD | 0,75 | 0,75 |
| Glycérine | 22,00 | 22,00 |
| Transcutol^{®} CG | 2,00 | 2,00 |
| E-Leen^{®} Green C | 1,50 | 1,50 |
| Eau déminéralisée | 52,75 | 52,75 |

Les résultats obtenus sont présentés au tableau 5 ci-dessous :

**Tableau 5**

| | **Composition selon l'invention** L-glutamine **micronisée** : taille volumique médiane des particules de L-glutamine inférieure à 15,0 microns (pourcentage en poids relatif au poids total de la composition) | | **Composition comparative** L-glutamine **cristalline** : taille volumique médiane des particules de L-glutamine de 154 microns (pourcentage en poids relatif au poids total de la composition) | |
|---|---|---|---|---|
| Texture | non abrasive | | abrasive | |
| Stabilité de la forme galénique | | - pas de déphasage de la forme galénique ; | | - déphasage de la forme galénique ; |
| | | - pas de relargage des cristaux de L-glutamine ; | | - relargage des cristaux de L-glutamine ; |
| | | | | - sédimentation de la L-glutamine. |
| | | - pas de sédimentation de la L-glutamine. | | |

Comme on peut le constater, une composition selon l'invention comprenant des particules de L-glutamine ayant une taille volumique médiane inférieure à 15,0 microns présente une texture adéquate pour une application topique tandis que la composition comparative comprenant de la L-glutamine sous forme cristalline ayant une taille volumique médiane de 154 microns est abrasive. Par ailleurs, une composition selon l'invention comprenant des particules de L-glutamine ayant une taille volumique médiane inférieure à 15,0 microns ne donne pas lieu à un déphasage de la forme galénique, ne donne pas lieu à un relargage des cristaux de L-glutamine et le donne pas lieu à la sédimentation de la L-glutamine, ceci au contraire de ce qui est observé avec la composition comparative comprenant de la L-glutamine sous forme cristalline ayant une taille volumique médiane de 154 microns.

## Revendications

1. Composition dermatologique [composition (D), ci-après] destinée à être utilisée par administration topique comprenant
une quantité dermatologiquement active de particules de L-glutamine ou l'un de ses sels pharmaceutiquement acceptables, et
au moins un excipient acceptable pour une administration topique,
dans laquelle la taille volumique médiane des particules de L-glutamine ou l'un de ses sels pharmaceutiquement acceptables est inférieure à 15,0 microns.

2. La composition dermatologique selon la revendication 1, dans laquelle la taille volumique médiane des particules de L-glutamine ou l'un de ses sels pharmaceutiquement acceptables est comprise entre 1,0 et 10,0 microns.

3. La composition dermatologique selon l'une quelconque des revendications 1 ou 2, comprenant relatif au poids total de la composition (D), de 1,0 à 10,0 % en poids desdites particules de L-glutamine ou l'un de ses sels pharmaceutiquement acceptables.

4. La composition dermatologique selon l'une quelconque des revendications précédentes, dans laquelle ledit au moins un excipient acceptable pour administration topique comprend l'acide oléique.

5. La composition dermatologique selon la revendication 4, comprenant relatif au poids total de la composition (D), de 2,0 à 20,0 % en poids d'acide oléique, de préférence de 5,0 à 10,0 % en poids d'acide oléique.

6. La composition dermatologique selon l'une quelconque des revendications précédentes, ladite composition étant sous la forme d'une solution, d'un gel, d'une lotion, d'une crème, d'un onguent, d'une mousse, d'une émulsion, d'une microémulsion, d'un lait, d'un sérum, d'un aérosol, d'une pulvérisation, d'un aérosol, d'une pulvérisation, d'une dispersion, ou d'un savon, de préférence sous la forme d'une crème.

7. Utilisation non-thérapeutique de la composition dermatologique selon l'une quelconque des revendications précédentes, pour favoriser la densification et/ou l'homogénéisation de l'épiderme, atténuer les rides, atténuer les vergetures, repulper et/ou raffermir la peau.

8. Procédé de traitement cosmétique de la peau destiné à favoriser la densification et/ou l'homogénéisation de l'épiderme, atténuer les rides, atténuer les vergetures, repulper et/ou raffermir la peau, comprenant l'application topique d'une composition dermatologique selon l'une quelconque des revendications 1 à 6.
